# EUROPEAN PATENT SPECIFICATION

(11) **EP 0 617 015 B1**
(45) Date of publication and mention of the grant of the patent: **29.10.1997**
(21) Application number: 94103360.7
(22) Date of filing: 05.03.1994
(51) Int. Cl.: C07C 401/00, C07F 9/535

(54) **Process for preparing vitamin D3 derivatives and intermediates thereof**
Verfahren zur Herstellung von Vitamin-D3-Derivaten und deren Zwischenprodukte
Procédé de préparation des dérivés de vitamine D3 ainsi que de leurs produits intermédiaires

(30) Priority: 18.03.1993 US 32905
(43) Date of publication of application: 28.09.1994
(73) Proprietor: F. HOFFMANN-LA ROCHE AG, 4002 Basel (CH)
(72) Inventor: Masami, Okabe, Nutley, New Jersey 07110 (US)
(74) Representative: Mahé, Jean

(56) References cited:
- EP-A- 0 154 185
- TETRAHEDRON LETTERS, vol. 34, no. 41, 1993, Oxford, GB, pages 6533-6536, M. OKABE et al, "An efficient synthesis of (22E,25R)-1alpha,25,26-trihydroxy-22ene-vitamin D3"
- PROC. WORKSHOP VITAMIN D, vol. 6, 1985, pages 755-764, P. M. WOVKULICH et al, "Synthesis of 1alpha-25,26-trihydroxy-22ene-cholecalciferol, a potent inducer of cell differentiation"
- BLOOD, vol. 74, no. 1, July 1989, pages 82-93, J. ZHOU et al, "Novel vitamin D analogs that modulate leukemic cell growth and differentiation with little effect on either intestinal calcium absorption or bone calcium mobilization"

## Description

The invention relates to a process for the preparation of the compounds 1α,25(R),26-trihydroxy-22-ene-cholecalciferol and 1α,25(S),26-trihydroxy-22-ene-cholecalciferol, represented by the formula

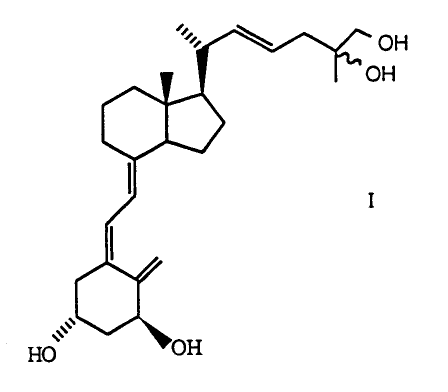

This process comprises
a) reacting a compound of the formula Ph₃P=CHLi, with a specific epimer of the formula 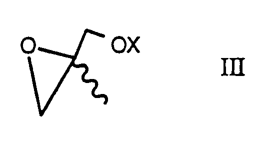 wherein X is an oxygen protecting group,
   to form the corresponding specific epimer of the formula 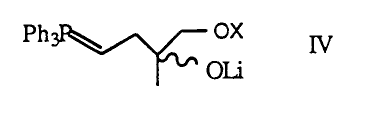
b) reacting the specific epimer of formula IV with a compound of the formula 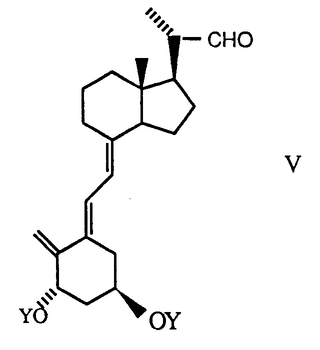 wherein Y is an oxygen protecting group,
   to form the corresponding specific epimer of the formula 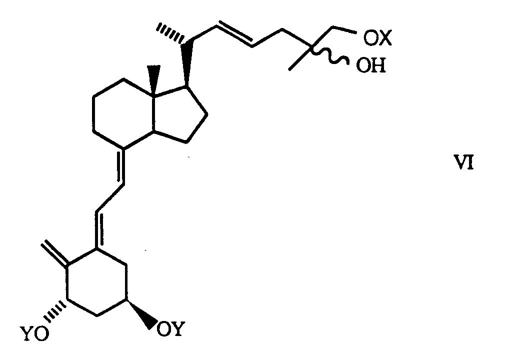 wherein X and Y are as described above,
c) converting the specific epimer of formula VI into the corresponding specific epimer of the formula 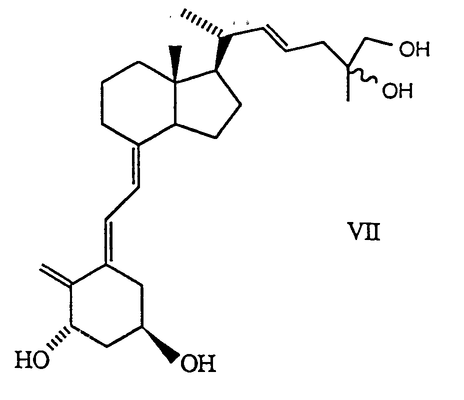
d) isomerizing the specific epimer of formula VII to form the corresponding specific epimer of formula I.

A process for preparing compounds of formula I is described in US 5 110 958. This process utilizes intermediates having the same 5Z, 7E configuration as the products of formula I. In the instant process the intermediates of formulae V to VII have the 5E, 7E configuration.

A preferred aspect of the invention is the reaction of a compound of formula IV with a compound of formula V to a compound of formula VI, wherein X and Y are silyl containing oxygen protecting groups.

It is understood that while the C-25 epimers are prepared and discussed individually, mixtures of the C-25 epimers are also within the scope of the invention. As used herein, the term "oxygen protecting group" denotes an ether or silyl ether group, such as tetrahydropyranyl or -Si(R¹,R²,R³), wherein R¹, R², and R³ are independently alkyl or phenyl, preferably a trialkylsilyl group. The term "alkyl" denotes a straight-chain or branched chain alkyl group containing 1 to 10, preferably 1 to 6, carbon atoms.

In accordance with the invention, a compound of the formula [Ph₃P-CH₃]⁺Z⁻, wherein Z is halogen other than fluorine, is treated with sec.butyllithium or tert.butyllithium in an aprotic organic solvent, preferably ether, to form a known compound of formula Ph₃P=CHLi, which is reacted with a specific epimer of formula III, a known compound or which can be prepared by known methods, to yield the corresponding specific epimer of formula IV. The reaction is carried out in an aprotic organic solvent, such as THF or preferably ether.

The specific epimer of formula IV is reacted with a compound of formula V, a known compound or which can be prepared by known methods, to form the corresponding specific epimer of formula VI. The reaction is carried out in an aprotic organic solvent, as mentioned above, preferably at a temperature in the range of -78° to 25°C.

The specific epimer of formula VI is treated with a fluoride salt, such as tetrabutylammonium fluoride in THF, to form the corresponding specific epimer of formula VII.

The specific epimer of formula VII is subjected to photo-isomerization, preferably using 9-acetylanthracene as the catalyst in methanol and tert.butyl methyl ether, to form the corresponding compound of formula I.

The compounds of the formula 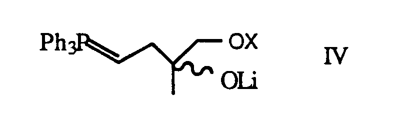 wherein X is an oxygen protecting group, particularly wherein X is -Si(R¹,R²,R³), R¹, R² and R³ being C₁-C₆ alkyl or phenyl, specially wherein X is thexyldimethylsilyl, as well as the compounds of the formula 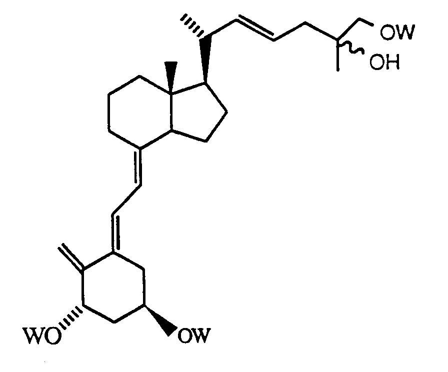 wherein W is hydrogen or the same or different oxygen protecting group, particularly tert.butyldimethylsilyl or thexyldimethylsilyl, specially the compounds of the group consisting of the following:
(1α,3β,5E,7E,22E,25R)-9,10-secocholesta-5,7,10(19),22-tetraene-1,3,25,26-tetrol,
(1α,3β,5E,7E,22E,25S)-9,10-secocholesta-5,7,10(19),22-tetraene-1,3,25,26-tetrol,
(1α,3β,5E,7E,22E,25R)-1,3-bis[[(1,1-dimethyl)dimethylsilyl]oxy]-26-[[dimethyl(1,1,2-trimethylpropyl)silyl]oxy]-9,10-secocholesta-5,7,10(19),22-tetraen-25-ol and
(1α,3β,5E,7E,22E,25S)-1,3-bis-[[(1,1-dimethyl)dimethylsilyl]oxy]-26-[[dimethyl(1,1,2-trimethylpropyl)silyl]oxy]-9,10-secocholesta-5,7,10(19),22-tetraen-25-ol,
are novel and constitute a further aspect of the invention.

The examples which follow further illustrate the invention.

### EXAMPLE 1

A suspension of 6.48 g of methyltriphenylphosphonium bromide and 65 ml of anhydrous ether was cooled under Ar to -30°C. Then, 30.9 ml of 1.3M sec.butyllithium in cyclohexane was added dropwise. Then, the mixture was stirred at room temperature for 4 hours. After the resulting orange suspension was cooled to 5°C, 7.11 g of (R)-dimethyl[(2-methyloxiranyl)methoxy] (1,1,2-trimethylpropyl)silane was added. After 20 minutes, the mixture was stirred at room temperature overnight. The yellow suspension was then cooled to -70°C, and 7.43 g of (1α,3β,5E,7E,20S)-1,3-bis[[(1,1-dimethylethyl)-dimethylsilyl]oxy]-9,10-secopregna-5,7,10(19)-triene-20-carboxaldehyde was added. The mixture was stirred at -70°C for 1 hour and then slowly warmed up to room temperature. The reaction mixture was cooled again to 5°C and quenched by the addition of 1.48 ml of acetic acid followed by addition of water. The mixture was extracted with ether. The combined organic layers were dried and concentrated. The residue was partitioned between hexane and 95% methanol. The hexane layer was washed with 95% methanol. The methanol layers were extracted with hexane. The hexane layers were dried and concentrated. The residue was suspended in methanol and the mixture was cooled to solidify the product. The resulting suspension was diluted with 95% methanol and stirred at 0°C prior to filtration. The solid was washed with 95% methanol and dried. The product was suspended in methanol, and the mixture was stirred at room temperature. After dilution with 95% methanol, the mixture was stirred at 0°C. The precipitate was filtered, washed with 95% methanol, and dried to give 7.38 g (71.0%) of (1α,3β,5E,7E,22E,25R)-1,3-bis[[(1,1-dimethylethyl)dimethylsilyl]oxy]-26-[[dimethyl(1,1,2-trimethyl-propyl)silyl]oxy]-9,10-secocholesta-5,7,10(19),22-tetraen-25-ol as a white solid: mp 82-86°C.

| | | | |
|---|---|---|---|
| Anal. Calcd. for C₄₇H₈₈O₄Si₃: | C, 70.44; | H, 11.07; | Si, 10.51. |
| Found: | C, 70.08; | H, 11.05; | Si, 10.24. |

### EXAMPLE 2

A solution of 7.38 g of the product of Example 1, and 92 ml of 1M tetrabutylammonium fluoride in THF was stirred under Ar at 30°C for 24 hours, and then poured into a stirred mixture of 300 ml of water and 100 ml of hexane. The resulting suspension was stirred at room temperature. The precipitate was filtered and washed with water and then with hexane. After drying, the solid was dissolved in methanol, and then diluted with water. After warming, the mixture was stored in a refrigerator. The precipitate was filtered and washed with 50% methanol. After drying, 3.23 g (81.5%) of (1α,3β,5E,7E, 22E,25R)-9,10-secocholesta-5,7,10(19),22-tetraene-1,3,25,26-tetrol was obtained as a white solid: mp 163-166°C.
Anal. Calcd. for C₂₇H₄₂O₄: C, 75.31; H, 9.83; Found: C, 75.06; H, 9.73.

### EXAMPLE 3

A solution of 3.20 g of the product of Example 2, 64 mg of 9-acetylanthracene, 1.2 l of methanol and 0.5 l of tert.butyl methyl ether was cooled to -20°C under Ar and irradiated with a 450 W medium pressure mercury lamp through a uranium glass filter for 3 hours. The solution was concentrated at room temperature to give a white solid, and it was suspended in ethyl acetate. After stirring at room temperature, the suspension was diluted with hexane, and stored in a refrigerator. The solid was filtered and washed with ethyl acetate-hexane (3:1). 3.11 g of the crude product thus obtained was dissolved in methanol at reflux. After cooling, the solution was diluted with water, and stirred at room temperature prior to filtration. The solid was washed with 50% methanol and dried to give 2.85 g (89.1%) of 1α,25R,26-trihydroxy-22E-ene-cholecalciferol as a white solid: mp 176-178°C.
Anal. Calcd. for C₂₇H₄₂O₄: C, 75.31; H, 9.83; Found: C, 75.06; H, 9.90.

## Claims

1. A compound of the formula 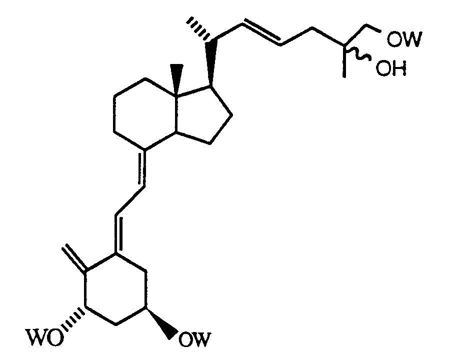 wherein W is hydrogen or the same or different oxygen protecting group, particularly tert.butyldimethylsilyl or thexyldimethylsilyl.

2. The compound of Claim 1, of the group consisting of the following:
(1α,3β,5E,7E,22E,25R)-9,10-secocholesta-5,7,10(19),22-tetraene-1,3,25,26-tetrol,
(1α,3β,5E,7E,22E,25S)-9,10-secocholesta-5,7,10(19),22-tetraene-1,3,25,26-tetrol,
(1α,3β,5E,7E,22E,25R)-1,3-bis[[(1,1-dimethyl)dimethylsilyl]oxy]-26-[[dimethyl(1,1,2-trimethylpropyl)silyl]oxy]-9,10-secocholesta-5,7,10(19),22-tetraen-25-ol and
(1α,3β,5E,7E,22E,25S)-1,3-bis[[(1,1-dimethyl)dimethylsilyl]oxy]-26-[[dimethyl(1,1,2-trimethylpropyl)silyl]oxy]-9,10-secocholesta-5,7,10(19),22-tetraen-25-ol.

3. A compound of the formula 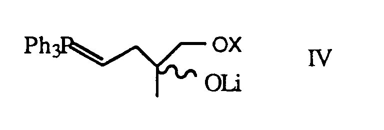 wherein X is an oxygen protecting group, particularly wherein X is -Si(R¹,R²,R³), R¹, R² and R³ being C₁-C₆ alkyl or phenyl, specially wherein X is thexyldimethylsilyl.

4. A process for preparing a specific epimer of the formula 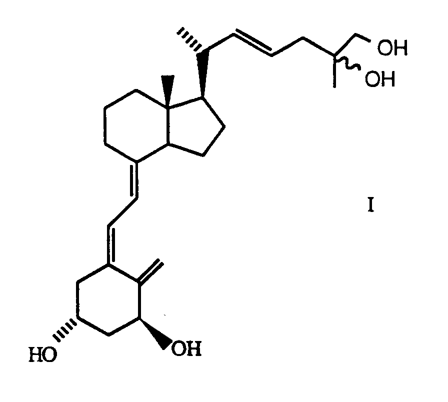 comprising
a) reacting a compound of the formula Ph₃P=CHLi, with a specific epimer of the formula 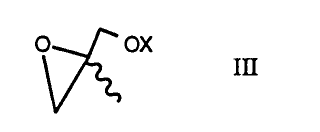 wherein X is an oxygen protecting group,
to form the corresponding specific epimer of the formula 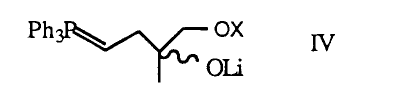
b) reacting the specific epimer of formula IV with a compound of the formula 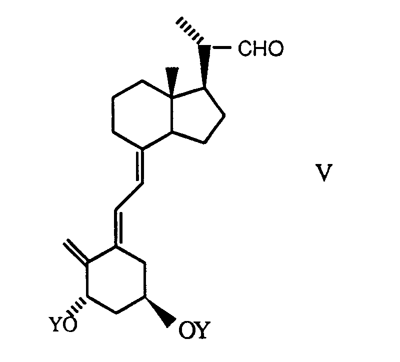 wherein Y is an oxygen protecting group,
to form the corresponding specific epimer of the formula 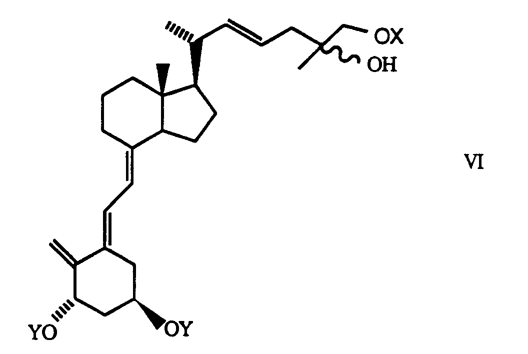 wherein X and Y are as described above,
c) converting the specific epimer of formula VI into the corresponding specific epimer of the formula 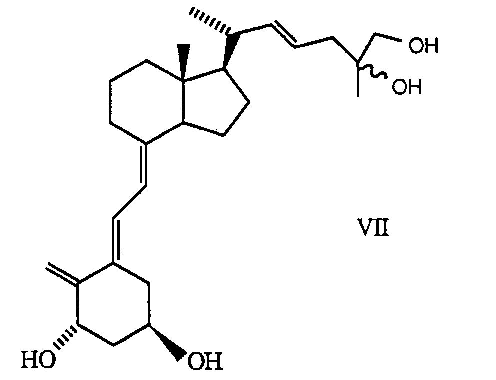
d) isomerizing the specific epimer of formula VII to form the corresponding specific epimer of formula I.

5. The process of Claim 4, wherein X is tetrahydropyranyl or -Si(R³,R⁴,R⁵), wherein R³, R⁴, R⁵ are independently alkyl or phenyl.

6. A process for preparing a compound of the formula 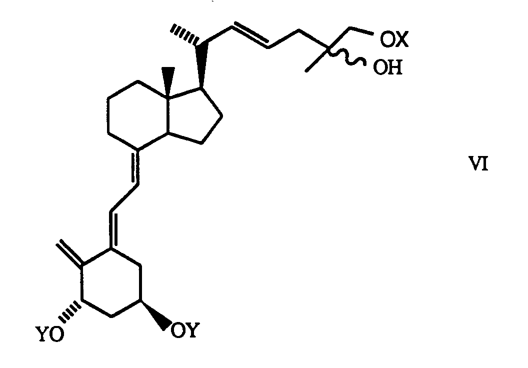 wherein X and Y are the same or different silyl containing oxygen protecting group,
comprising reacting a compound of the formula 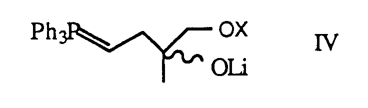 wherein X is a silyl containing oxygen protecting group, with a compound of the formula 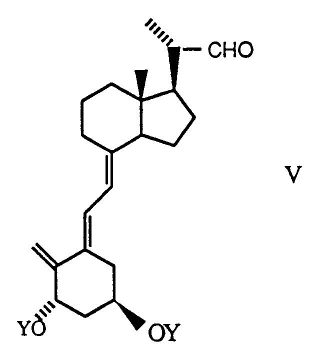 wherein Y is as described above.

## Patentansprüche

1. Verbindung der Formel 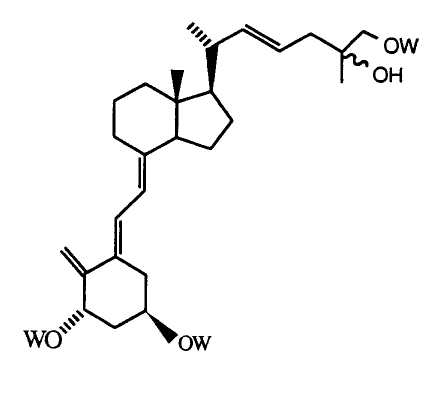 worin W Wasserstoff oder die gleichen oder verschiedene Sauerstoffschutzgruppen, insbesondere t-Butyldimethylsilyl oder Thexyldimethylsilyl, ist.

2. Die Verbindungen gemäss Anspruch 1 aus der Gruppe der folgenden:
(1α,3β,5E,7E,22E,25R)-9,10-Secocholesta-5,7,10(19),22-tetraen-1,3,25,26-tetrol,
(1α,3β,5E,7E,22E,25S)-9,10-Secocholesta-5,7,10(19),22-tetraen-1,3,25,26-tetrol,
(1α,3β,5E,7E,22E,25R)-1,3-bis[[(1,1-Dimethyl)dimethylsilyl]oxy]-26-[[dimethyl(1,1,2-trimethylpropyl)silyl]oxy]-9,10-secocholesta-5,7,10(19),22-tetraen-25-ol und
(1α,3β,5E,7E,22E,25S)-1,3-bis[[(1,1-Dimethyl)dimethylsilyl]oxy]-26-[[dimethyl(1,1,2-trimethylpropyl)silyl]oxy]-9,10-secocholesta-5,7,10(19),22-tetraen-25-ol.

3. Verbindung der Formel 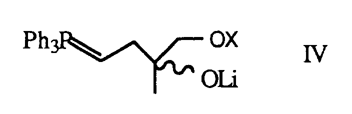 worin X eine Sauerstoffschutzgruppe, insbesondere worin X die Gruppe -Si (R¹, R², R³) ist und R¹, R² und R³ C₁-C₆-Alkyl oder Phenyl sind, speziell worin X Thexyldimethylsilyl ist.

4. Verfahren zur Herstellung eines bestimmten Epimers der Formel 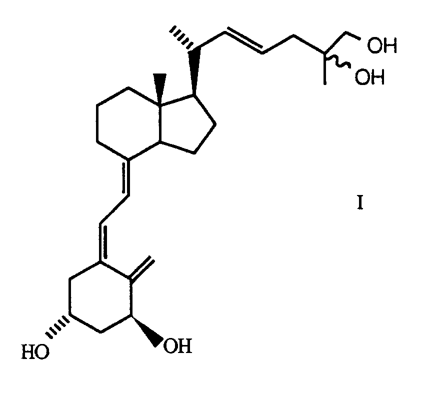 wobei man
a) eine Verbindung der Formel Ph₃P=CHLi mit einem bestimmten Epimer der Formel 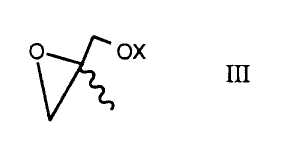 worin X eine Sauerstoffschutzgruppe ist,
unter Bildung des entsprechenden bestimmten Epimers der Formel 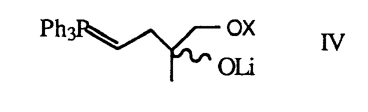 umsetzt,
b) den bestimmten Epimer der Formel IV mit einer Verbindung der Formel 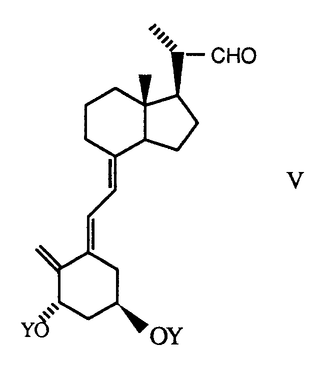 worin Y eine Sauerstoffschutzgruppe ist,
unter Bildung des entsprechenden bestimmten Epimers der Formel 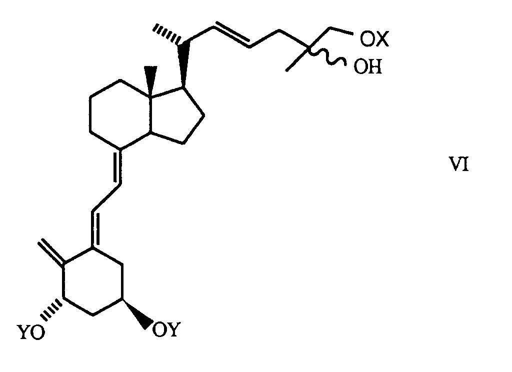 worin X und Y obige Bedeutung haben,
umsetzt,
c) den bestimmten Epimer der Formel VI in den entsprechenden bestimmten Epimer der Formel 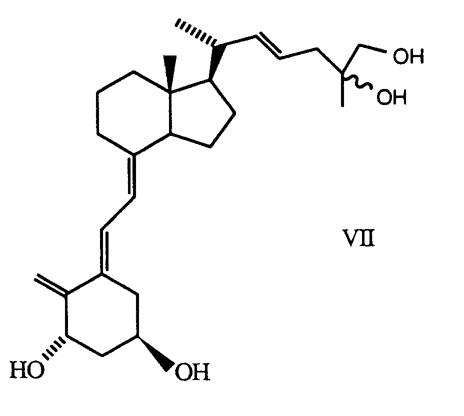 überführt,
d) den bestimmten Epimer der Formel VII unter Bildung des entsprechenden bestimmten Epimers der Formel I isomerisiert.

5. Verfahren gemäss Anspruch 4, worin X Tetrahydropyranyl oder -Si(R³,R⁴,R⁵) ist und R³, R⁴ und R⁵ unabhängig voneinander Alkyl oder Phenyl sind.

6. Verfahren zur Herstellung einer Verbindung der Formel 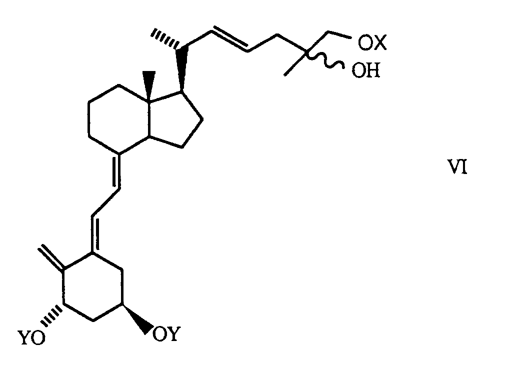 worin X und Y die gleichen oder verschiedene Silyl enthaltenden Sauerstoffschutzgruppen sind,
wobei man eine Verbindung der Formel 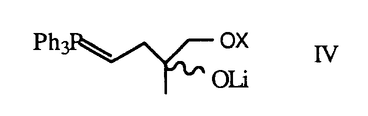 worin X eine Silyl enthaltende Sauerstoffschutzgruppe ist,
mit einer Verbindung der Formel 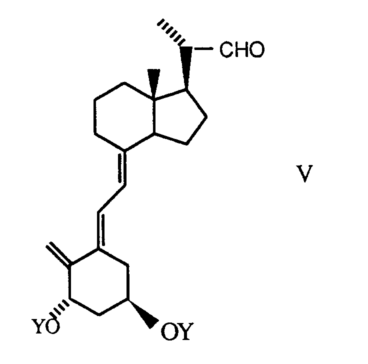 worin Y obige Bedeutung hat,
umsetzt.

## Revendications

1. Composé de formule 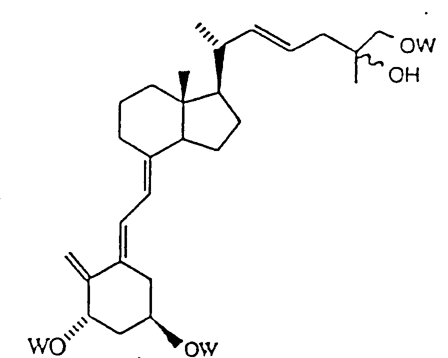 dans laquelle W représente un atome d'hydrogène ou des groupes protecteurs d'atome d'oxygène identiques ou différents, en particulier le groupe tert-butyldiméthylsilyle ou thexyldiméthylsilyle.

2. Composé selon la revendication 1, appartenant au groupe constitué par les composés suivants:
(1α,3β,5E,7E,22E,25R)-9,10-sécocholesta-5,7,10(19),22-tétraène-1,3,25,26-tétrol,
(1α,3β,5E,7E,22E,25S)-9,10-sécocholesta-5,7,10(19),22-tétraène-1,3,25,26-tétrol,
(1α,3β,5E,7E,22E,25R)-1,3-bis[[(1,1-diméthyl)diméthylsilyl]oxy]-26-[[diméthyl(1,1,2-triméthylpropyl)silyl]oxy]-9,10-sécocholesta-5,7,10(19),22-tétraène-25-ol et
(1α,3β,5E,7E,22E,25S)-1,3-bis[[(1,1-diméthyl)diméthylsilyl]oxy]-26-[[diméthyl(1,1,2-triméthylpropyl)silyl]oxy]-9,10-sécocholesta-5,7,10(19),22-tétraène-25-ol.

3. Composé de formule 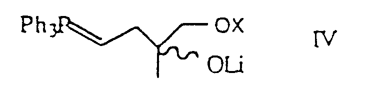 dans laquelle X est un groupe protecteur d'atome d'oxygène, en particulier dans laquelle X est un groupe -Si(R¹,R²,R³), R¹, R² et R³ étant chacun un groupe alkyle en C₁-C₆ ou phényle, en particulier dans laquelle X est le groupe thexyldiméthylsilyle.

4. Procédé pour la préparation d'un épimère déterminé de formule 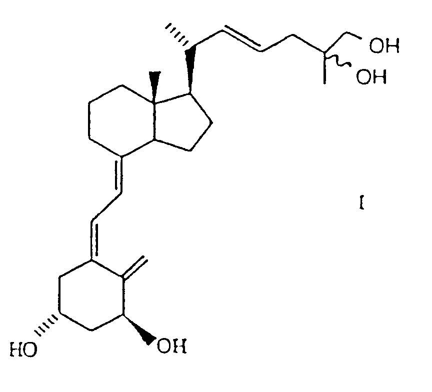 comprenant
a) la mise en réaction d'un composé de formule Ph₃P=CHLi avec un épimère déterminé de formule 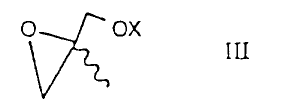 dans laquelle X est un groupe protecteur d'atome d'oxygène, pour la formation de l'épimère déterminé correspondant de formule 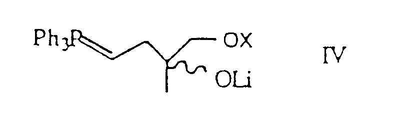
b) la mise en réaction de l'épimère déterminé de formule IV avec un composé de formule 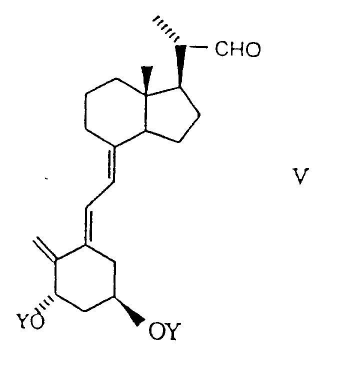 dans laquelle Y est un groupe protecteur d'atome d'oxygène, pour la formation de l'épimère déterminé de formule 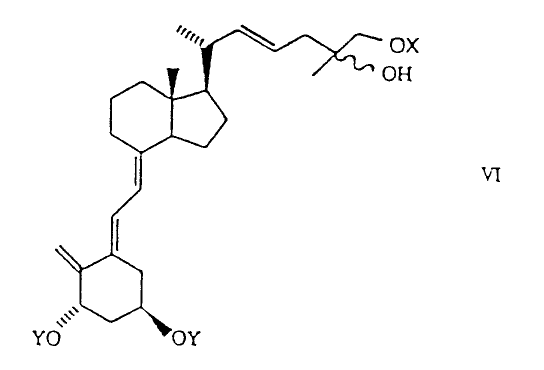 dans laquelle X et Y sont tels que décrits plus haut,
c) la conversion de l'épimère déterminé de formule VI en l'épimère déterminé correspondant, de formule 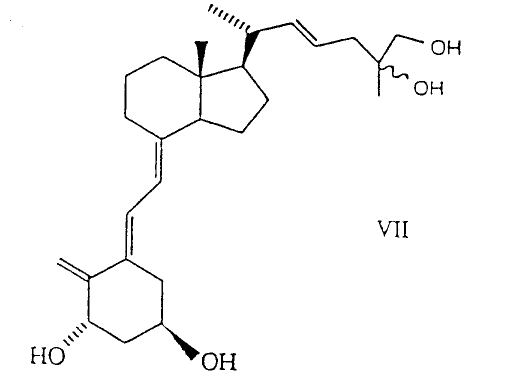
d) l'isomérisation de l'épimère déterminé de formule VII, pour la formation de l'épimère déterminé correspondant de formule I.

5. Procédé selon la revendication 4, dans laquelle X est le groupe tétrahydropyrannyle ou -Si(R³,R⁴,R⁵), R³, R⁴, R⁵ étant indépendamment un groupe alkyle ou phényle.

6. Procédé pour la préparation d'un composé de formule 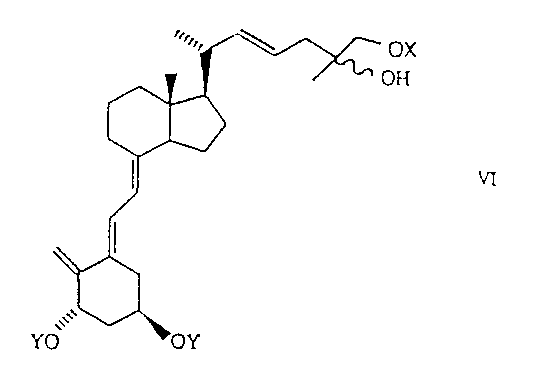 dans laquelle X et Y représentent des groupes protecteurs d'atome d'oxygène silylés, identiques ou différents, comprenant la mise en réaction d'un composé de formule 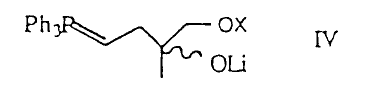 dans laquelle X est un groupe protecteur d'atome d'oxygène, contenant le radical silyle,
avec un composé de formule 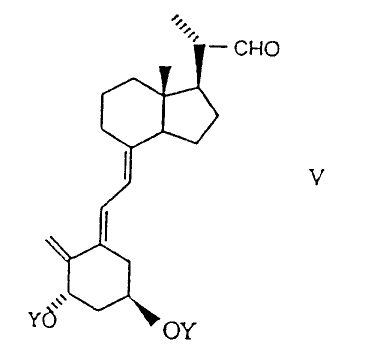 dans laquelle Y est tel que décrit plus haut.
